# EUROPEAN PATENT APPLICATION

(11) **EP 0 573 682 A1**
(43) Date of publication of application: **15.12.1993**
(21) Application number: 92109657.4
(22) Date of filing: 09.06.1992
(51) Int. Cl.: A61K 31/35, A61K 35/78

(54) **Tea polyphenols as anti-hyperglycemic agents**

(71) Applicant: MITSUI NORIN CO., LTD., Tokyo (JP)
(72) Inventor: Haro,Yukihiko, Shizuoka-ken (JP); Tona-Oka,Fumiko, Shizuoka-ken (JP); Ishigaki,Akiyo, Shizuoka-ken (JP); Matsumoto,Natsuki, Fujieda-shi,Shizuoka-ken (JP)
(74) Representative: VOSSIUS & PARTNER

(57) **Abstract**

A method for preparing a pharmaceutical composition for treating hyperglycemia, comprising administering to a patient in thereof an effective amount of tea polyphenol. Anti-hyperglycemic agents of this invention have no harmful side effects to the human body.

## Description

The present invention relates to a method of treating hyperglycemia and, more precisely, to a method of treating hyperglycemia with anti-hyperglycemic agent containing tea polyphenols as an active ingredient.

It has been discovered that particular glycopeptides and oligosaccharides derived from microorganisms have an anti-hyperglycemic action. However, since these substances may have some harmful side-effects wherein a large dosage or continuous dosage may have a detrimental effect on the human body, they have not yet been put to practical use.

One of the present inventors previously found that tea polyphenols have an inhibiting activity on α-amylase, a digestive enzyme which hydrolyzes polysaccharides, and on the basis of the finding, filed a patent application,

An object of the present invention is to provide a method of treating hyperglycemia and, to develop an agent which has effective anti-hyperglycemic activity and may be used without the fear of harmful side-effects to the human body.

One of the present inventors conducted various research with the purpose of finding natural substances with pharmaceutical efficacy. Having already established a method of efficiently extracting tea polyphenols from tea leaves, they used this method to isolate tea polyphenols and made repeated studies on their physiological activities. As a result, tea polyphenols were found to have excellent hyperglycemic activity. On the basis of this finding, the present invention was achieved.

### BRIEF EXPLANATION OF THE DRAWINGS

Fig. 1 shows time-dependent variation of glucose concentration in plasma when crude mixture of tea catechins (Trade name: Polyphenon100, product of Mitsui Norin Co. Ltd.) was administered.

Fig. 2 shows time-dependent variation of insulin concentration in plasma on the same condition as above.

Fig. 3 shows time-dependent variation of glucose concentration in plasma when green tea was administered on the same conditon as above.

Fig. 4 shows time-dependent variation of glucose concentration in plasma when black tea was administered on the same condition as above.

### DETAILED DESCRIPTION OF THE INVENTION

The present invention relates to a method for preparing a pharmaceutical composition for treating hyperglycemia. An effective amount of a composition comprising at least one tea polyphenol selected from the group consisting of (-)epicatechin gallate, (-)epigallocatechin gallate, free theaflavin, theaflavin monogallate B and theaflavin digallate is administered to a patient in need thereof.

Tea polyphenols include tea catechins of the following general formula (1) and tea theaflavins of the following general formula (2). 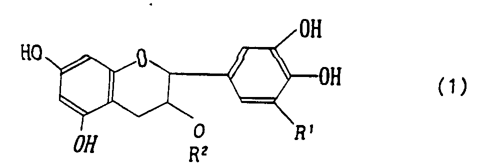
Where R¹ represents H or OH; and R² represents H or 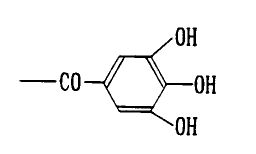
Examples of catechins of the formula (1) are as follows:
(-) Epicatechin (in the formula (1), R¹=H, R²=H)
(-) Epigallocatechin (in the formula (1), R¹=OH, R²=H)
(-) Epicatechin gallate (in the formula (1), R¹=h, R²= 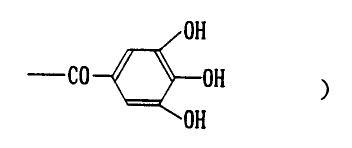
(-) Epigallocatechin gallate (in the formula (1), R¹=H, R²= 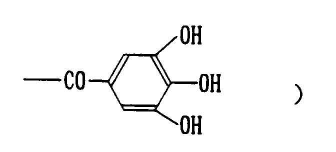
Polyphenol 100 (Trade name, product of Mitsui Norin Co. Ltd.) which will be mentioned hereinafter is a mixture of the above-mentioned four kinds of tea catechins. 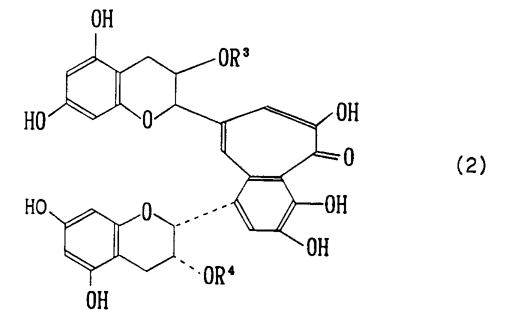
where R³ and R⁴ each represent H or 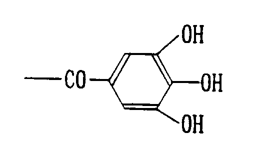
and R³ and R⁴ may be the same or different from each other.

Examples of theaflavins of the formula (2), are as follows:
free theaflavin (in the formula (2), R³=H, R⁴=H)
theaflavin monogallate A (in the formula (2), R³= 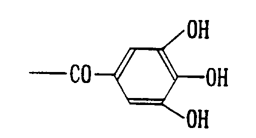
R₄=H)
theaflavin monogallate B (in the formula (2), R³=H, R⁴= 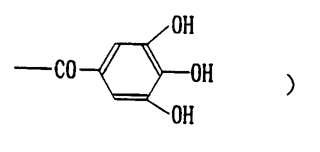
theaflavin digallate (in the formula(2), R³=R⁴= 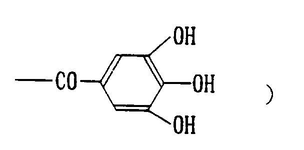
The above-mentioned tea polyphenols can be produced from the raw material, tea leaves, and the method of producing them is described in U.S. patents Nos. 4,613,672, 4,673,530, 4,913,909.

Where the anti-hyperglycemic agent of the present invention is administered as a drug to the human body, the active main component of the agent, tea polyphenols, should be used in an amount displaying sufficient anti-hyperglycemic activity. In general, peroral administration ranging from approximately 100 mg to 5 g of the main component a day is preferred.

The anti-hyperglycemic agent of the present invention is used for medicinal purposes or can be added to food etc.; the main component of the agent, tea polyphenols, may be used or added thereto directly or in the form of a solution dissolved in water or alcohols such as ethyl alcohol, glycerol, propylene glycol, etc. If desired, it may be combined with a suitable carrier.

The present invention will be explained in more detail by way of the following examples, which, however, are not intended to restrict the scope of the invention.

### EXAMPLE 1

Wistar rats were given no food overnight and 80mg of Polyphenon 100 (Trade name, product of Mitsui Norin Co., Ltd.) was administered 30 minutes prior to administration of 4ml of aqueous 40% soluble starch solution. This group of rats was the test group. The control group was given no food overnight and perorally administered with 4ml of aqueous 40% soluble starch solution only.

Immediately after the administration and 30 minutes, 1 hour, 2 hours and 4 hours after the administration, blood was taken from the heart of each rat with a syringe to which heparin had been added, and each of the blood samples was subjected to centrifugation (3000 rpm, 20 minutes) to obtain plasma therefrom.

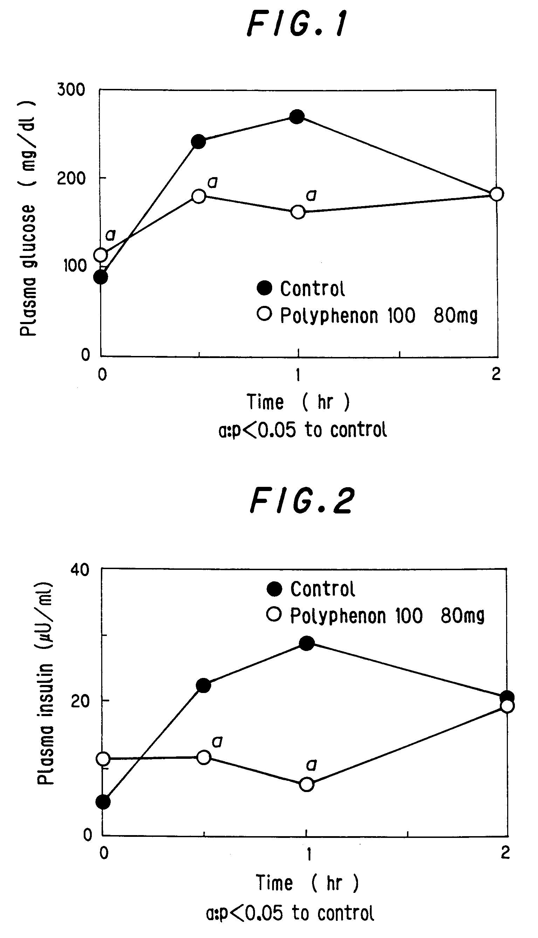

Glucose in the thus obtained plasma sample was measured by a enzymatic method, and insulin therein was measured by a one step enzyme immunoassay method. The thus measured glucose and insulin concentration in the blood are shown in Fig. 1 and Fig. 2, respectively.

As is obvious from these drawings, the blood glucose level of the rats of the control group was about 100mg/dl immediately after administration of soluble starch thereto and it increased up to about 250mg/dl in 30 minutes levelling out to about 200mg/dl thereafter. In the same way, blood insulin level of control group increased to more than 20 µU/ml in 30 minutes and peaked out around 30 µU/ml in 1 hour.

Though both the blood glucose level and the blood insulin level of the rats of the test group were not so different from those of the rats of the control group immediately after administration of soluble starch and Polyphenon 100 thereto, the blood glucose level of the former was about 170mg/dl in 30 minutes and the blood insulin level of the same was about 10 µU/ml in 30 minutes. That is, in 30 minutes after administration, both the blood sugar level and the blood insulin level of rats of the test group were significantly lower than those of the control group.

From these results, it is obvious that administration of a polyphenol along with starch results in suppression of the rising blood glucose level.

### EXAMPLE 2

(-)Epigallocatechin gallate was tested with respect to its acute toxicity. Where (-)epigallocatechin gallate was perorally administered to 6 week ICR male mice, LD₅₀ after one week was 2314mg/kg. Where (-)epigallocatechin was administered intraperitonealy to 5 week old female ICR mice, LD₅₀ after one week was 150mg/kg.

### EXAMPLE 3

| Composition of Tablets: | |
|---|---|
| Crude Catechin (or (-)epigallocatechin gallate) | 100 mg |
| Light Silicic Acid Anhydride | 80 mg |
| Crystalline Cellulose | 140 mg |
| Lactose | ad lib. |
| Magnesium Stearate | 2 mg |

The above ingredients were formed into tablets using a conventional method. One tablet contains the above-stated quantities.

### Example 4

Japanese green tea was extracted with hot water and freeze dried. More or less of 0.4g of this dried matter dissolved in 100ml of hot water would correspond to a daily cup of green tea of normal pungency. This sample is referred to as "green tea" hereinafter.

Indian black tea extract powder was prepared in the same manner. More or less of 0.4g of this dried powder dissolved in 100ml of hot water would probably correspond to a daily cup of tea of nomal pungency without milk or sugar. This sample is referred to as "black tea" hereinafter.

A male Wistar strain of rats, 6 weeks of age were divided into 4 groups of 5 rats and fed a commercial diet (Oriental Yeast Co., Ltd.) for a week. The room temperature was controlled at 23±2°C and lighting was on a 12 hour cycle (0700-1900 light).

The rats were given no food overnight and 80mg/ml green tea extract powder solution (1 ml) or black tea extract powder solution (1 ml) were administered orally to the test groups. The control groups were given water (1 ml). After 30 minutes, 40% soluble starch (4 ml) was administered orally to rats of all groups. The blood of each rat was collected immediately after, 30 minutes, 1 hour and 2 hours after administration of starch. Concentrations of glucose in the blood were determined.

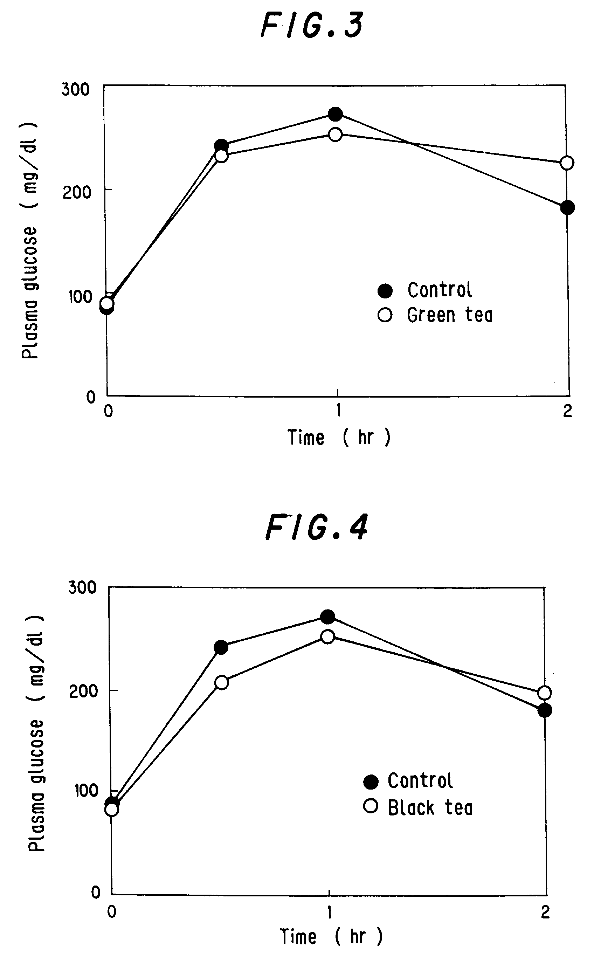

Results showed that neither green tea nor black tea suppressed the elevation of plasma glucose levels caused by the administration of starch (Fig 3, 4). Both of the samples consisted of tea and therefore contain tea polyphenols, however ordinary tea was not found to be effective in the way that Polyphenon 100 in Example 1 was in suppressing blood glucose levels. As many as 13 cups worth of soluble tea at each meal will not suppress the glucose levels in the blood, whereas concentrated tea polyphenols of the same weight or less are extremely effective in suppressing blood glucose levels.

As has been explained in detail in the above, since tea polyphenol used in the method of the present invention is a natural substance which is commonly consumed in fairly large amount, it may be used not only for medicinal purposes but also as an additive to foods without fear of harmful side effects to human body. In addition, even though a small amount of the tea polyphenol is taken, one may obtain a sufficient anti-hyperglycemic effect therefrom.

## Claims

1. Use of at least one tea polyphenol selected from the group consisting of (-)epicatechin gallate, (-)epigallocatechin gallate, free theaflavin, theaflavin monogallate A, theaflavin monogallate B and theaflavin digallate for the preparation of a pharmaceutical agent for treating hyperglycemia.

2. Use as claimed in claim 1 wherein tea polyphenols are used together with at least one pharmaceutically acceptable carrier.

3. Use as claimed in claim 1 wherein said pharmaceutical agent is administered to give 100 mg to 5 grams of said tea polyphenol per day.

4. A pharmaceutical composition for treating hyperglycemia comprising an effective amount of at least one tea polyphenol as defined in claim 1 and optionally at least one pharmaceutically acceptable carrier.

5. Use of at least one tea polyphenol as described in claim 1 as food additives for non-medicinal purposes.

6. Use according to claim 4 for the preparation of a dietary food product.

7. A food composition for dietary purposes suited to lower the blood glucose level comprising an effective amount of at least one tea polyphenol as defined in claim 1.
